(19) Europäisches Patentamt
European Patent Office
Office européen des brevets

(11) EP 0 601 406 B1

(12) EUROPÄISCHE PATENTSCHRIFT

(45) Veröffentlichungstag und Bekanntmachung des Hinweises auf die Patenterteilung:
**28.02.1996 Patentblatt 1996/09**

(51) Int. Cl.[6]: **C07C 315/04**, C07C 317/36

(21) Anmeldenummer: **93118988.0**

(22) Anmeldetag: **25.11.1993**

(54) **Verfahren zur Herstellung von (5-Amino-2-(2-hydroxyethylamino)phenyl)(2-hydroxyethyl)sulfon**

Method for the preparation of (5-amino-2-(2-hydroxyethylamino)phenyl)(2-hydroxyethyl) sulphone

Procédé pour la préparation du (5-amino-2-(2-hydroxyéthylamino)phényl)(2-hydroxyéthyl) sulfone

(84) Benannte Vertragsstaaten:
**CH DE FR GB LI**

(30) Priorität: **08.12.1992 DE 4241284**

(43) Veröffentlichungstag der Anmeldung:
**15.06.1994 Patentblatt 1994/24**

(73) Patentinhaber: **BAYER AG**
**D-51368 Leverkusen (DE)**

(72) Erfinder:
- **Herd, Karl-Josef, Dr.**
  **D-51519 Odenthal (DE)**
- **Henk, Hermann, Dr.**
  **D-51061 Köln (DE)**

(56) Entgegenhaltungen:
**EP-A- 0 431 389**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 601 406 B1

Printed by Rank Xerox (UK) Business Services
2.9.9/3.4

**Beschreibung**

[5-Amino-2-(2-hydroxyethylamino)phenyl](2-hyxdroxyethyl)sulfon der Formel (1) ist ein wichtiges Zwischenprodukt in der Farbstoffsynthese, insbesondere in der Reaktivfarbstoffsynthese, wie es z.B. in DE-A 35 12 340 oder US-A-4 577 015 beschrieben ist.

In US-A-5 107 025 wird ein Herstellungsverfahren beschrieben, das auf der katalytischen Reduktion von Monoazo/azoxy-Verbindungen basiert. Nicht optimal gestaltet sich nach diesem Verfahren die Abtrennung von (1) und der jeweiligen wiedereinzusetzenden Diazokomponente. Entscheidenden Einfluß darauf hat das Löslichkeitsprodukt des Betains der Diazokomponente in Wasser.

Gegenstand der Erfindung ist deshalb ein neues Verfahren zur Herstellung von [5-Amino-2-(2-hydroxyethylamino)phenyl](2-hydroxyethyl)sulfon der Formel (1)

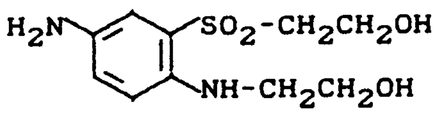

(1)

dadurch gekennzeichnet, daß man Disazo/Disazoxyfarbstoffe der Formel (5)

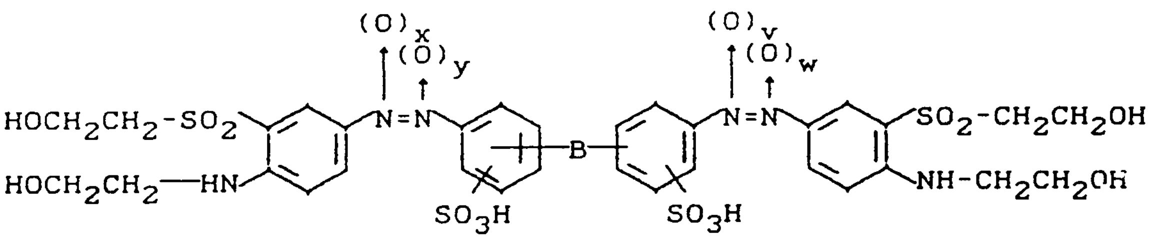

(5)

worin

B = direkte Bindung oder $CH_2$, $CH_2CH_2$, $SO_2$, CO, O, CH=CH, NHCONH, $OCH_2CH_2O$, NH oder N($C_1$-$C_4$-Alkyl), vorzugsweise $CH_2CH_2$ oder CH=CH, und

v,w,x,y = unabhängig voneinander 0 oder 1,

wobei $x + y = 0$ oder 1 und $v + w = 0$ oder 1 ,

reduktiv in die Diaminoverbindung der Formel (2)

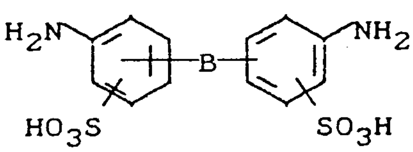

(2)

und in 2 Äquivalente der Formel (1) spaltet und die dabei entstehende Diaminoverbindung der Formel (2) nach Sauerstellen und Ausfällen als doppeltes, in Wasser gering lösliches Betain abtrennt, wobei unter gering löslichem Betain solches mit einer Löslichkeit von 0,01 g/l bis 1 g/l (25° C) verstanden wird.

Das nahezu quantitativ ausgefällte und abgetrennte (2) wird wieder in die Synthese eingesetzt. Die in Lösung verbleibende Verbindung der Formel (1) kann entweder durch Aufkonzentrieren isoliert oder aber direkt in dieser Form weiter umgesetzt werden.

Die Reduktion der Disazo/Disazoxy-Farbstoffe der Formel (5) zu (1) und (2) kann nach Methoden erfolgen, wie sie in US-A-5 107 025 sowie von R. Schröter im Handbuch für präparative Methoden der organischen Chemie, Houben-Weyl, Band XI, Teil 1, Seite 522 bis 531 beschrieben werden. Als besonders vorteilhaft erweist sich danach eine Reduktion mit Natriumdithionit oder Traubenzucker oder aber eine katalytische Reduktion mit Wasserstoff. Als Katalysatoren kommen dafür insbesondere Raney-Nickel, Palladium-Kohle oder Platinverbindungen in Frage. Die Reduktion wird bevorzugt in Wasser bei Temperaturen zwischen 20 und 80° C und unter pH-neutralen Bedingungen, bevorzugt zwischen pH 5 bis pH 8, durchgeführt. Die katalytische Reduktion mit Wasserstoff wird im allgemeinen bei Drucken von 10 bis 100 atm, vorzugsweise von 30 bis 80 atm, insbesondere bei 50 bis 70 atm in einem dafür geeigneten Autoklaven durchgeführt.

In einer besonderen Ausführungsform des Verfahrens werden Disazo/Disazoxy-Farbstoffe der Formel (5a)

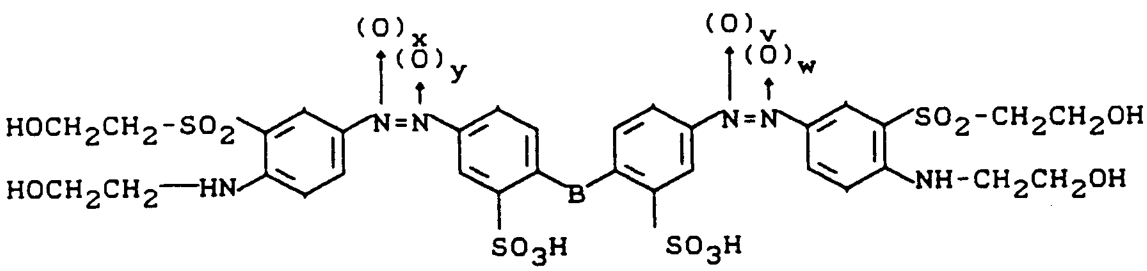

(5a)

worin

B = $CH_2CH_2$ oder CH=CH und
v,w,x,y = unabhängig voneinander 0 oder 1,

wobei v + w = 0 oder 1 und x + y = 0 oder 1
zu (1) und Diaminoverbindungen der Formel (2a)

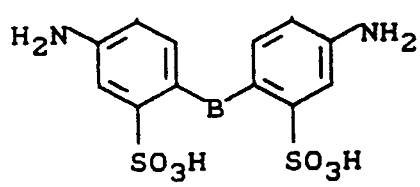

(2a)

reduziert.

In einer weiteren besonderen Ausführungsform des Verfahrens werden die Verbindungen der Formel (1) durch Reduktion der Disazo/Disazoxy-Farbstoffe der Formel (5) erhalten, die ihrerseits dadurch erhalten werden können, daß man Diaminoverbindungen der Formel (2)

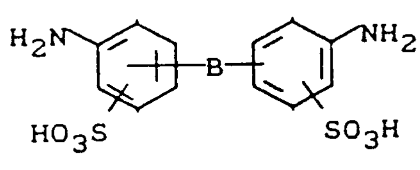

(2)

worin

B = direkte Bindung oder. $CH_2$, $CH_2CH_2$, $SO_2$, CO, O, CH=CH, NHCONH, $OCH_2CH_2O$, NH oder N($C_1$-$C_4$-Alkyl)

tetrazotiert, die Tetrazoniumverbindungen auf 2 Äquivalente 2-(2-Hydroxyethyl)mercapto-N-(2-hydroxyethyl)anilin der Formel (3)

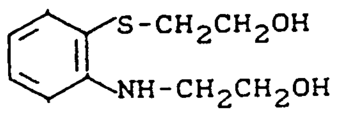

(3)

kuppelt und die resultierenden Disazoverbindungen der Formel (4)

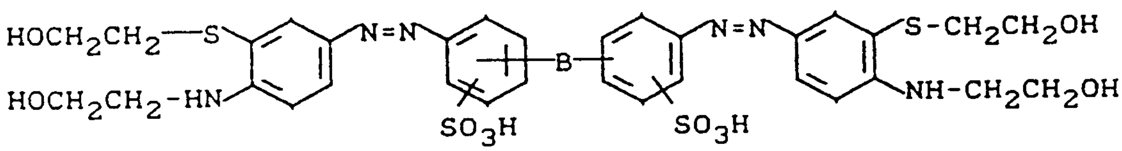

(4)

zu Disazo/Disazoxy-Farbstoffen der Formel (5) oxidiert

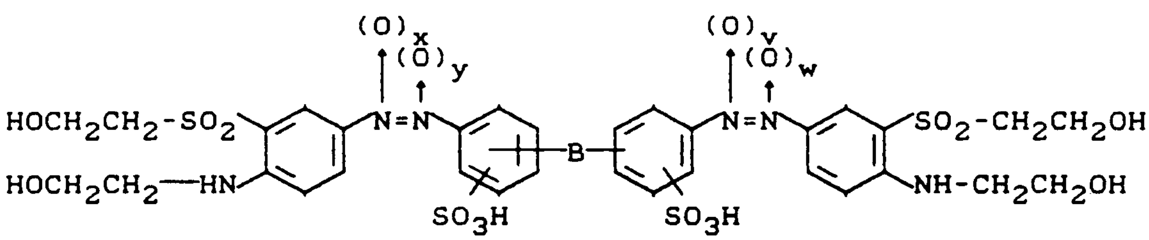

(5)

worin

v,w,x und y = unabhängig voneinander 0 oder 1,

wobei v + w = 0 oder 1 und
x + y = 0 oder 1 .

Eine besondere Ausführungsform dieses Verfahrens besteht darin, daß man Diaminoverbindungen der Formel (2a)

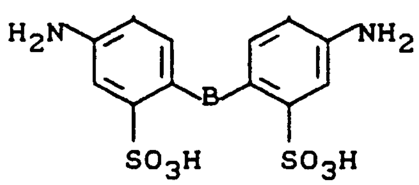

(2a)

worin

B = $CH_2$-$CH_2$ oder CH=CH,

einsetzt. Das doppelte Betain von (2a) besitzt nur eine geringe Wasserlöslichkeit von etwa 0,5 g/l bei 25 °C.

Sulfogruppenhaltige Diaminoverbindungen bieten den Vorteil, daß das Verfahren, d.h. Diazotierung, Kupplung, Oxidation und Reduktion, in wäßrigem Reaktionsmedium durchgeführt werden kann. Die Azokupplung auf 2 Äquivalente

(3) mit tetrazotierten Diaminoverbindungen (2) erfolgt vorzugsweise bei Temperaturen zwischen 0 und 30° C und zwischen pH-Werten von vorzugsweise 0,5 bis 5,0. Hierbei kann sich der Einsatz von Emulgatoren oder Kupplungsbeschleunigern, wie z.B. Harnstoff, als vorteilhaft erweisen. Die Farbstoffe (4) fallen als kristalline Verbindungen an und können deshalb durch Filtration isoliert werden. Da die Kupplungsreaktion meist relativ einheitlich verläuft, kann auch direkt ohne Zwischenisolierung von (4) zu Disazo/Disazoxy-Farbstoffen (5) oxidiert werden.

Als Oxidationsmittel können beispielsweise Wasserstoffperoxid, Perborate, Persulfate, Persulfonsäuren oder Peroxide der Alkali- und Erdalkalimetalle gegebenenfalls in Gegenwart geeigneter Katalysatoren verwendet werden.

In einer besonders bevorzugten Ausführungsform des Verfahrens hat sich Wasserstoffperoxid als Oxidationsmittel in Gegenwart katalytischer Mengen (0,001 bis 2 Gew.-% bezogen auf (4)) an Wolframaten und/oder Vanadaten, insbesondere Alkali-Wolframaten und/oder -Vanadaten, als vorteilhaft erwiesen.

Die Oxidation erfolgt im allgemeinen in wäßrigem Medium bei 20 bis 100° C, vorzugsweise bei 40 bis 90° C, und bei pH-Werten von 4 bis 8. Dabei werden als Zwischenstufen die entsprechenden Sulfoxidverbindungen durchlaufen.

Die Disazo/Disazoxy-Farbstoffe (5) sind als gelbe bis rote, kristalline Verbindungen isolierbar und können zusätzlich durch Umkristallisieren aus Wasser bzw. Wasser/Alkohol-Mischungen gereinigt werden.

Nach der Reduktion resultiert eine Zweikomponentenlösung, aus der nach Abtrennen des Katalysators die Diaminoverbindung (2) durch Ansäuern mit Mineralsäuren, wie z.B. Schwefelsäure oder Salzsäure, zur Fällung gebracht und durch Filtration oder Abpressen nahezu quantitativ als doppeltes Betain abgetrennt werden kann. Die Verbindung (1) verbleibt im sauren Filtrat und kann nun isoliert oder nach Neutralisation direkt als Lösung weiter umgesetzt werden. Die abgetrennte Diaminoverbindung (2) kann nach Tetrazotierung erneut in die nächste Synthesesequenz eingesetzt werden.

Die angegebenen Formeln für die Zwischenprodukte und Disazofarbstoffe sind die der freien Säuren. Bei der Herstellung werden im allgemeinen die Salze eingesetzt bzw. erhalten, insbesondere die Alkalisalze, wie beispielsweise Na-, K- oder Li-Salze.

Beispiel 1

54 g 4,4'-Diaminostilben-2,2'-disulfonsäure werden in 300 ml Wasser, 100 g Eis und 60 ml konz. Salzsäure verrührt und danach durch Zugabe von 70 ml einer 30 Vol%igen wäßrigen Natriumnitritlösung tetrazotiert. Die Suspension wird 1 Stunde bei 5° C gerührt. Der Nitritüberschuß wird durch Zugabe von Amidosulfonsäure nach Reaktionsende entfernt. Dazu fügt man nun eine Lösung 65 g 2-(2-Hydroxyethylmercapto)-N-(2-hydroxyethyl)anilin in 50 ml Wasser/15 ml konz. Salzsäure. Nach 30 Minuten stellt man bei ca. 10° C den pH-Wert mit Sodalösung innerhalb 1 Stunde von 0,5-0,8 auf pH 1,5 und läßt 2 Stunden rühren. Anschließend rührt man 6 bis 8 Stunden bei 10 bis 15° C und pH 2,0 bis 2,5. Das ausgefallene Zwischenprodukt der Struktur

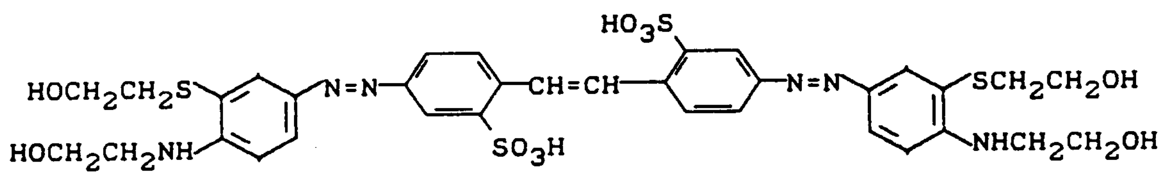

($\lambda_{max}$ = 481 nm) wird durch Abnutschen isoliert, und die feuchte Paste wird direkt weiter umgesetzt. Es kann aber auch auf eine Zwischenisolierung verzichtet werden und die Kupplungsmischung direkt oxidiert werden.

Zur Oxidation suspendiert man die feuchte Paste in 400 ml Wasser, stellt mit Sodalösung auf pH 5 und fügt 0,2 g Natriumwolframat zu. Es wird auf 60° C erwärmt. Ohne weitere Wärmezufuhr dosiert man nun langsam ca. 100 ml einer 30%igen wässrigen Wasserstoffperoxidlösung zu, wobei die Temperatur aufgrund der Reaktionswärme auf ca. 70 bis 75° C ansteigt, und eine Lösung resultiert. Während der Reaktion hält man den pH-Wert mit Sodalösung konstant bei 5,0. Nach einer Nachrührphase von 1 Stunde bei 75° C kühlt man langsam auf 20° C ab, isoliert den ausgefallenen Farbstoff und trocknet. Es werden ca. 100 g salzhaltiges Produkt der Struktur

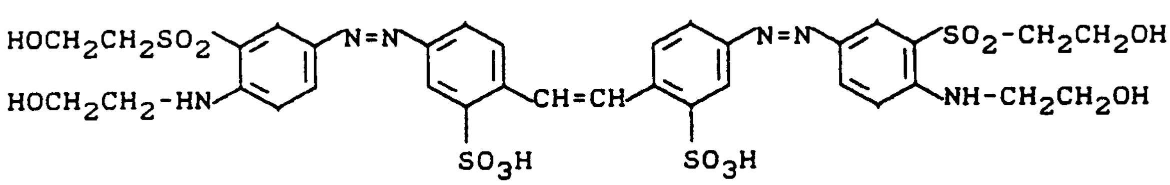

erhalten ($\lambda_{max}$ = 440 nm ($H_2O$)), wobei die angegebene Struktur mögliche Disazoxy-Verbindungen mit einschließt. Das Trocknen kann auch entfallen und die feuchte Farbstoffpaste direkt weiter umgesetzt werden, indem man sie in 250 ml Wasser bei pH 7 suspendiert, mit 0,2 g Raney-Nickel-Katalysator versetzt und im Autoklaven bei 50 bis 70 atm mit der 4,5 bis 4,7-fachen äquimolaren Menge Wasserstoff reduziert.

Die Suspension kann sich dabei auf 40 bis 50° C erwärmen. Nach Entspannen und Abtrennen des Katalysators durch Filtration resultiert eine klare, blaß-bräunliche Lösung. Es wird auf 60°C erwärmt und mit verdünnter Schwefelsaure die Lösung auf pH 1,0 gestellt. Dabei kristallisiert die 4,4'-Diaminostilben-2,2'-disulfonsäure nahezu quantitativ als Betain aus. Es wird in der Wärme abgenutscht und mit 50 ml kaltem Wasser gewaschen. Nach dem Trocknen erhält man ca. 40 g 4,4'-Diaminostilben-2,2'-disulfonsäure.

Das mit dem Waschwasser vereinte Filtrat enthält das gewünschte [5-Amino-2-(2-hydroxyethylamino)phenyl](2-hydroxyethyl)sulfon der Formel

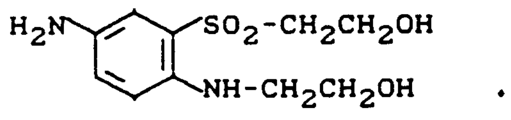

Die Gehaltsbestimmung der 350 ml Lösung erfolgt mittels Diazotierung. Danach sind ca. 55 g Produkt der obigen Formel in Lösung. Aufgrund eines DC-Vergleichs erweist sich diese Verbindung mit einer Substanzprobe, die nach dem Verfahren aus EP 153 599 hergestellt wurde, als identisch. Die obige Lösung kann nun nach Neutralisation für die Farbstoffsynthese eingesetzt werden.

Beispiel 2

54 g 2,2'-(1,2-Ethandiyl)bis[5-aminobenzolsulfonsäure] werden in 300 ml Wasser, 100 g Eis und 60 ml konz. Salzsäure verrührt und danach durch Zugabe von 70 ml einer 30 Vol%igen wäßrigen Natriumnitritlösung tetrazotiert. Die Suspension wird 1 Stunde bei 5° C gerührt. Der Nitritüberschuß wird durch Zugabe von Amidosulfonsäure nach Reaktionsende entfernt. Dazu fügt man nun eine Lösung 65 g 2-(2-Hydroxyethylmercapto)-N-(2-hydroxyethyl)anilin in 50 ml Wasser/15ml konz. Salzsäure. Nach 30 Minuten stellt man bei ca. 10° C den pH-Wert mit Sodalösung innerhalb 1 Stunde von 0,5 bis 0,8 auf pH 1,5 und läßt 2 Stunden rühren. Anschließend rührt man 6 bis 8 Stunden bei 10 bis 15°C und pH 2,0 bis 2,5. Das ausgefallene Zwischenprodukt der Struktur

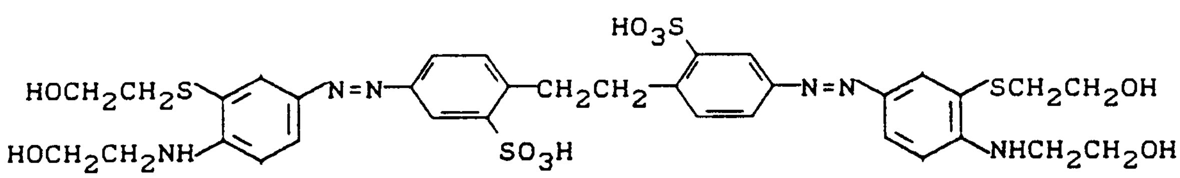

($\lambda_{max}$ = 440 nm) wird durch Abnutschen isoliert, und die feuchte Paste wird direkt weiter umgesetzt. Es kann aber auch auf eine Zwischenisolierung verzichtet werden und die Kupplungsmischung direkt nach folgender Vorschrift oxidiert werden:

Zur Oxidation suspendiert man die feuchte Paste in 400 ml Wasser, stellt mit Sodalösung auf pH 6,5 und fügt 0,2 g Natriumwolframat zu. Es wird auf 60°C erwärmt. Ohne weitere Wärmezufuhr dosiert man nun langsam ca. 100 ml einer 30%igen wässrigen Wasserstoffperoxidlösung zu, wobei die Temperatur aufgrund der Reaktionswärme auf ca. 70 bis 75°C ansteigt, und eine Lösung resultiert. Während der Reaktion hält man den pH-Wert mit Sodalösung konstant bei 5,0. Nach einer einstündigen Nachrührphase bei 75°C kühlt man auf 20°C ab, isoliert den ausgefallenen Farbstoff nach Aussalzen und trocknet. Es werden ca. 100 g salzhaltiges Produkt der Struktur

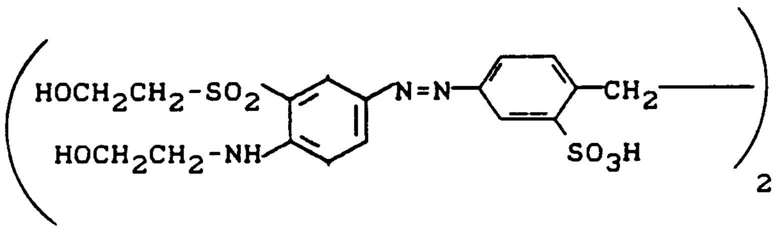

erhalten ($\lambda_{max}$ = 395, 435 (sh) nm ($H_2O$)), wobei die angegebene Struktur mögliche Disazoxy-Verbindungen mit einschließt.

Das Trocknen kann auch entfallen und die feuchte Paste kann direkt weiter umgesetzt werden.

Die feuchte Farbstoffpaste wird in 250 ml Wasser suspendiert, mit 0,2 g Raney-Nickel-Katalysator versetzt und im Autoklaven bei 50 bis 70 atm mit der 4,5 bis 4,7-fachen äquimolaren Menge Wasserstoff reduziert.

Die Suspension kann sich dabei exotherm auf 40°C erwärmen. Der Katalysator wird nach Reaktionsende abfiltriert und die verbleibende wäßrige Lösung auf 80°C erwärmt. Mit verdünnter Schwefelsäure stellt man den pH-Wert auf 1,0, wobei die 2,2'-(1,2-Ethandiyl)bis[5-aminobenzolsulfonsäure] nahezu quantitativ als doppeltes Betain ausfällt. Bei 60°C wird abgesaugt und mit 50 ml kaltem Wasser nachgewaschen. Die abgetrennte Diaminoverbindung kann nach Tetrazotierung erneut in die nächste Synthesesequenz eingesetzt werden.

Das mit dem Waschwasser vereinte Filtrat enthält das gewünschte [5-Amino-2-(2-hydroxyethylamino)phenyl]-(2-hydroxyethyl)sulfon, das nun, wie in Beispiel 1 beschrieben, analysiert und umgesetzt werden kann.

**Patentansprüche**

1. Verfahren zur Herstellung von [5-Amino-2-(2-hydroxyethylamino)phenyl](2-hydroxyethyl)sulfon der Formel (1)

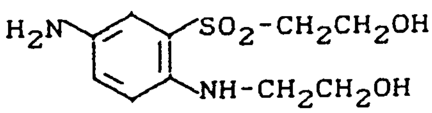

(1)

dadurch gekennzeichnet, daß man Disazo/Disazoxy-Farbstoffe der Formel (5)

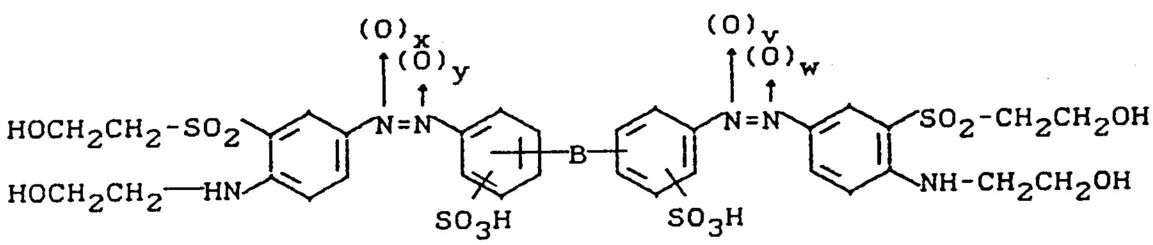

(5)

worin

B = direkte Bindung oder $CH_2$, $CH_2CH_2$, $SO_2$, CO, O, CH=CH, NHCONH, $OCH_2CH_2O$, NH oder N($C_1$-$C_4$-Alkyl) und

v, w, x und y = unabhängig voneinander 0 oder 1,

wobei x + y = 0 oder 1 und v + w = 0 oder 1 , reduktiv spaltet und die dabei entstehende Diaminoverbindung der

Formel (2)

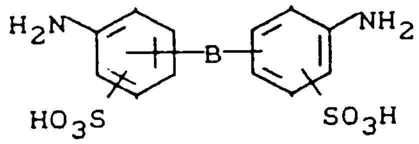

(2)

nach Sauerstellen und Ausfällen als doppeltes, in Wasser geringlösliches Betain abtrennt.

2. Verfahren gemäß dem Anspruch 1, dadurch gekennzeichnet, daß man Disazo/Disazoxy-Farbstoffe der Formel (5) mit B = $CH_2CH_2$ oder CH=CH einsetzt.

3. Verfahren gemäß wenigstens einem der Ansprüche 1 und 2, dadurch gekennzeichnet, daß man Disazo/Disazoxy-Farbstoffe der Formel (5a)

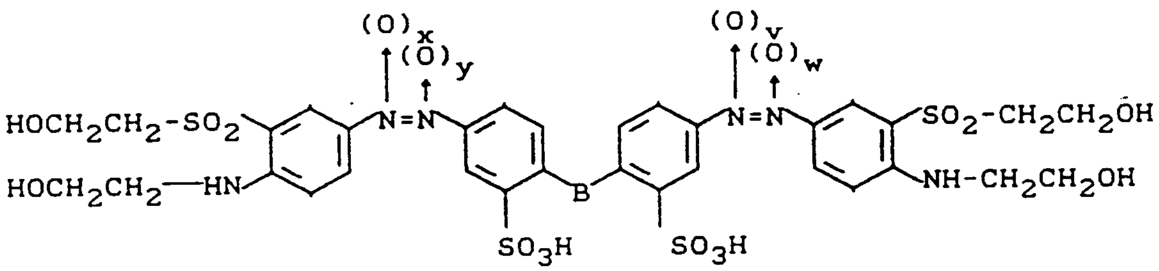

(5a)

worin B = $CH_2CH_2$ oder CH=CH und v,w,x und y die oben genannten Bedeutungen haben, einsetzt.

4. Verfahren gemäß Anspruch 1, dadurch gekennzeichnet, daß die Disazo/Disazoxy-Farbstoffe der Formel (5) erhalten werden, indem man Diaminoverbindungen der Formel (2)

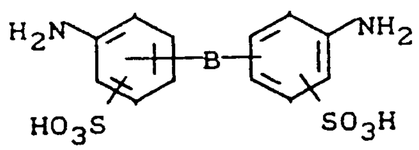

(2),

tetrazotiert, die Tetrazoniumverbindung auf 2 Äquivalente 2-(2-Hydroxyethyl)mercapto-N-(2-hydroxyethyl)anilin der Formel (3)

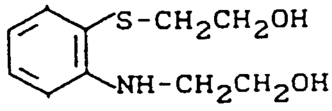

(3)

kuppelt, die resultierenden Disazoverbindungen der Formel (4)

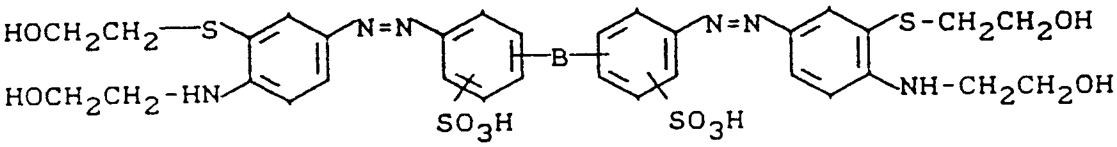

(4)

zu Disazo/Disazoxy-Farbstoffe der Formel (5)

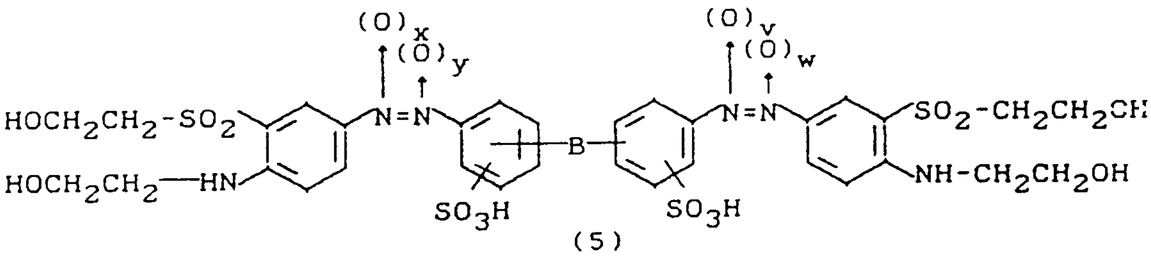

(5)

oxidiert, worin B, v, w, x und y die in Anspruch 1 angegebenen Bedeutungen haben.

**5.** Verfahren gemäß Anspruch 4, indem man Diaminoverbindungen der Formel (2a)

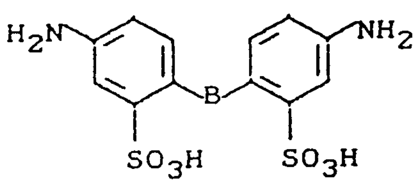

(2a)

mit B = $CH_2$-$CH_2$ oder CH=CH
einsetzt.

## Claims

1. Process for preparing 5-amino-2-(2-hydroxyethylamino)phenyl 2-hydroxyethyl sulphone of the formula (1)

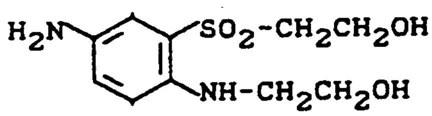

(1)

characterized in that disazo/disazoxy dyestuffs of the formula (5)

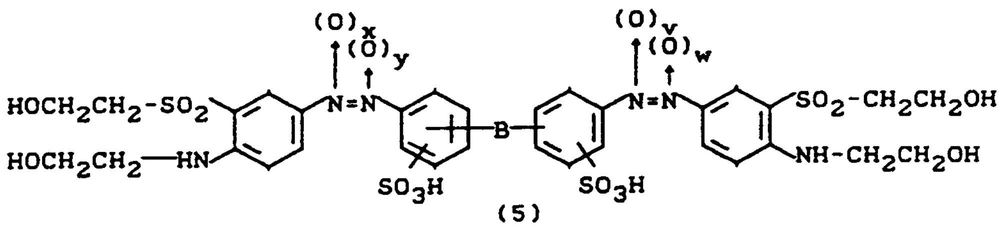

(5)

in which

B is a direct bond or $CH_2$, $CH_2CH_2$, $SO_2$, CO, O, CH=CH, NHCONH, $OCH_2CH_2O$, NH or N($C_1$-$C_4$-alkyl) and

v, w, x and y, independently of one another, are 0 or 1,
x + y being 0 or 1 and v + w being 0 or 1, are reductively cleaved, and the resulting diamino compound of the formula (2)

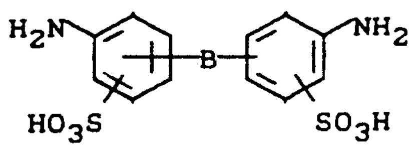

(2)

after acidification and precipitation, is separated off as a doubled, sparingly water-soluble betaine.

2. Process according to Claim 1, characterized in that disazo/disazoxy dyestuffs of the formula (5) where B is $CH_2CH_2$ or CH=CH are used.

3. Process according to at least one of Claims 1 and 2, characterized in that disazo/disazoxy dyestuffs of the formula (5a)

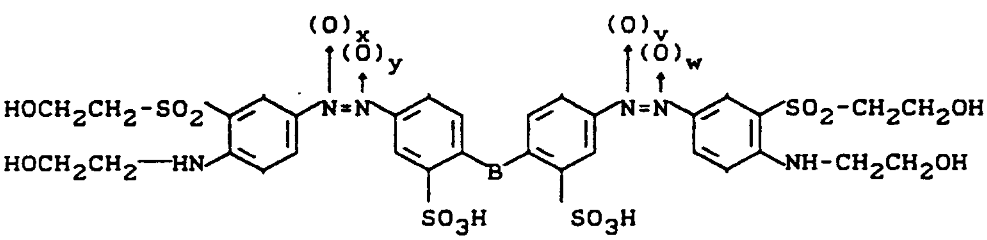

(5a)

in which B is $CH_2CH_2$ or CH=CH and v, w, x and y have the abovementioned meanings are used.

**4.** Process according to Claim 1, characterized in that the disazo/disazoxy dyestuffs of the formula (5) are obtained by tetrazotizing diamino compounds of the formula (2)

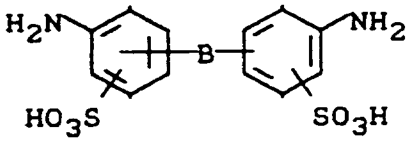

(2)

coupling the tetrazonium compound onto 2 equivalents of 2-(2-hydroxyethyl)mercapto-N-(2-hydroxyethyl)aniline of the formula (3)

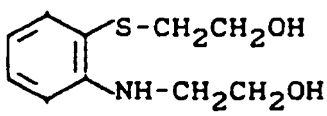

(3)

oxidizing the resulting disazo compounds of the formula (4)

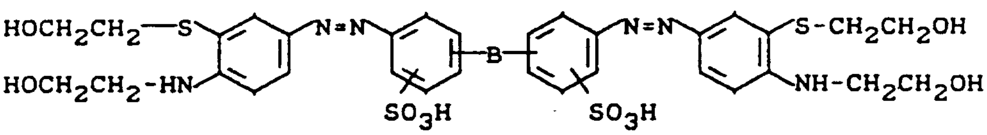

(4)

to disazo/disazoxy dyestuffs of the formula (5)

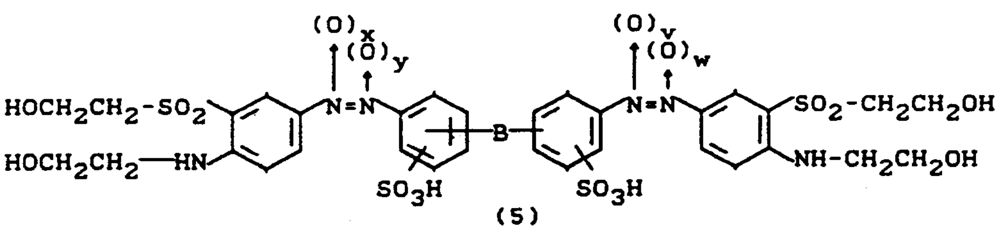

(5)

in which B, v, w, x and y have the meanings mentioned in Claim 1.

**5.** Process according to Claim 4, using diamino compounds of the formula (2a)

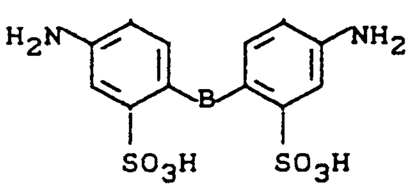

(2a)

where B is $CH_2$-$CH_2$ or CH=CH.

## Revendications

1. Procédé de préparation de la [5-amino-2-(2-hydroxyéthylamino)phényl](2-hydroxyéthyl)sulfone de formule (1) :

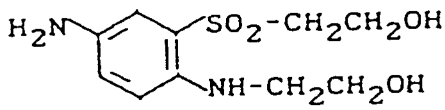

(1)

caractérisé en ce que l'on clive par réduction des colorants disazo/disazoxy de formule (5) :

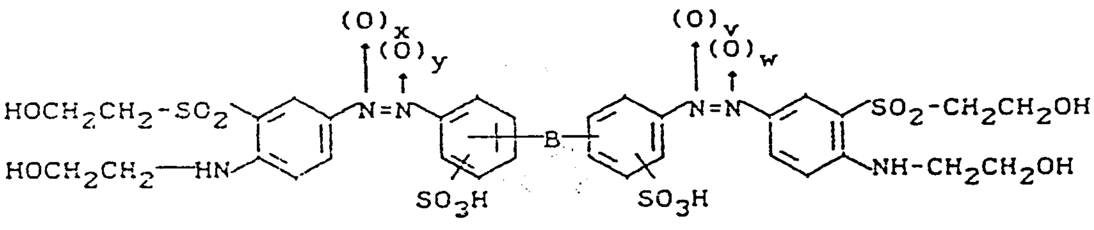

(5)

dans laquelle
B = liaison directe ou $CH_2$, $CH_2CH_2$, $SO_2$, CO, O, CH=CH, NHCONH, $OCH_2CH_2O$, NH ou N(alkyle en $C_1$-$C_4$), et
v, w, x et y = indépendamment les uns des autres 0 ou 1,
où x + y = 0 ou 1 et v + w = 0 ou 1 ,
et on sépare le composé diamino ainsi formé de formule (2) :

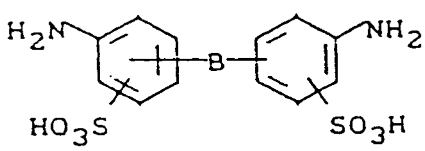

(2)

sous forme de bétaïne double faiblement soluble dans l'eau, après acidification et précipitation.

2. Procédé selon la revendication 1, caractérisé en ce que l'on utilise des colorants disazo/disazoxy de formule (5) avec B = $CH_2CH_2$ ou CH=CH.

3. Procédé selon au moins l'une des revendications 1 et 2, caractérisé en ce que l'on utilise des colorants disazo/disazoxy de formule (5a) :

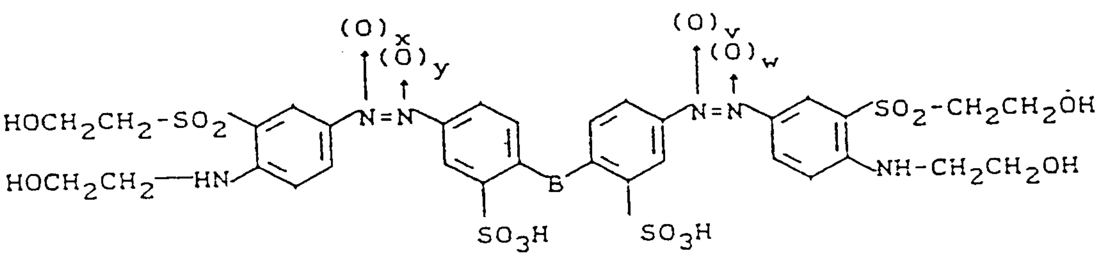

(5a)

dans laquelle

B = $CH_2CH_2$ ou CH=CH et
v, w, x et y ont les significations citées ci-dessus.

**4.** Procédé selon la revendication 1, caractérisé en ce que les colorants disazo/disazoxy de formule (5) sont obtenus en ce que l'on soumet des composés diamino de formule (2) :

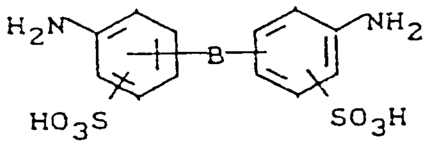

(2)

à une tétraazotation, on couple le composé de tétrazonium à 2 équivalents de 2-(2-hydroxyéthyl)mercapto-N-(2-hydroxyéthyl)aniline de formule (3) :

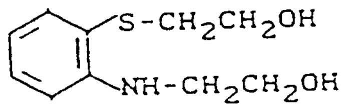

(3)

on oxyde les composés disazo de formule (4) résultants :

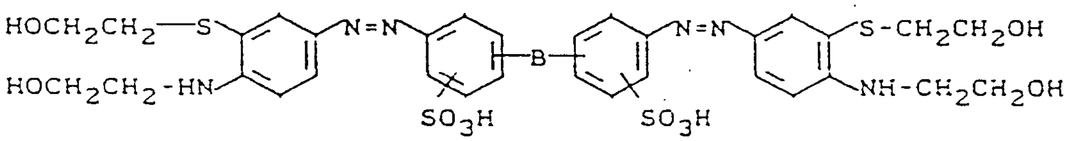

(4)

en colorants disazo/disazoxy de formule (5) :

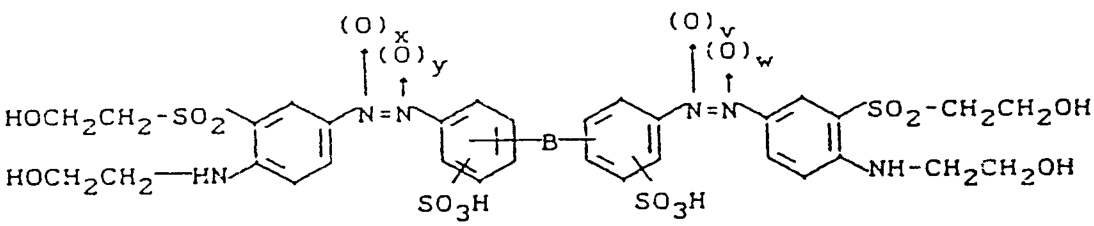

(5)

où B, v, w, x et y ont les significations indiquées dans la revendication 1.

**5.** Procédé selon la revendication 4, dans lequel on utilise des composés diamino de formule (2a) :

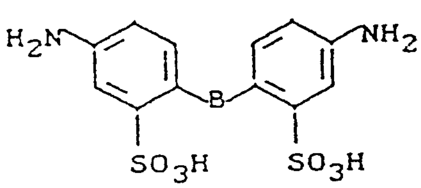

(2a)

avec B = $CH_2$-$CH_2$ ou CH=CH.